# EUROPEAN PATENT SPECIFICATION

(11) **EP 3 085 458 B1**
(45) Date of publication and mention of the grant of the patent: **25.10.2017**
(21) Application number: 16159480.9
(22) Date of filing: 24.05.2012
(51) Int. Cl.: B05B 7/10, B05B 13/02, B05B 7/00, A61M 5/32, B05D 5/08, B05B 7/04, B05B 7/12, B05D 1/02

(54) **DEVICE AND METHOD FOR COATING ELONGATE OBJECTS**
VORRICHTUNG UND VERFAHREN ZUM BESCHICHTEN LÄNGLICHER OBJEKTE
DISPOSITIF ET PROCÉDÉ DE REVÊTEMENT D'OBJETS ALLONGÉS

(30) Priority: 24.05.2011 US 201113114300
(43) Date of publication of application: 26.10.2016
(62) Divisional of application: 12169382.4
(73) Proprietor: NORDSON CORPORATION, Westlake, OH 44145-1119 (US)
(72) Inventor: Döker, Andreas, 82256 Fürstenfeldbruck (DE); Wanka, Thomas, 87600 Kaufbeuren (DE); Wilczek, Michael, 82110 Germering (DE)
(74) Representative: Eisenführ Speiser

(56) References cited:
- US-A- 5 628 826
- US-A1- 2010 203 232
- US-A1- 2011 111 116

## Description

### Technical Field

The present invention generally relates to the application of coatings onto exterior surfaces of objects and, more specifically, the coating of elongate objects such as needles.

### Background

Elongate objects are coated with material for various reasons. For example, hypodermic needles are often coated with lubricious or friction-reducing materials, such as silicone based oils. This provides a low friction outer surface of the needle for purposes of easing the introduction of the needle through the skin of a patient. Various methods and apparatus for applying the coating material to the needle have been used in the past. These include dipping methods in which the needles are dipped lengthwise into a bath containing silicone and solvent. This method presents difficulties and challenges related to handling the silicone and solvents, as well as the lengthy drying times involved, and the ventilation needs due to the use of the solvents.

Other methods that have been employed in the past involve various manners of spraying the coating material onto the needle. Typically, the spraying device will discharge the coating material in a single direction and the material will not flow around the entire exterior of the needle. For this reason, the needle and/or spray dispenser must be rotated to evenly distribute the coating material on the entire exterior surface of the needle. Alternatively, multiple spraying devices may be used to coat all sides of the needle. Either case involves increased complication and expense. The overspray or mist can also present problems in the environment around the equipment or require apparatus for containing the overspray. Due to the viscosity of the fluid coating material, it can also be difficult to achieve the required thin, uniform layer of coating material on the exterior surface of the needle.

US 2011/0111116 A1 discloses a swirl coating machine for swirl coating of a surgical device. The machine comprises opposed aligned spray nozzles which are provided for applying swirl coating to a plurality of surgical needles arranged side by side on a carrier strip. The swirl coating is applied during relative movement between the carrier strip and the spray nozzles. Other devices and methods for coating elongated objects are known from patent documents US 2005/0238829; US 5 628 826 and US 2010/203232.

It would therefore be desirable to provide a device and method for easily coating an exterior of an elongate workpiece, such as a needle, while addressing various challenges presented by past devices and methods.

### Summary

The present invention generally provides a device for coating an exterior of an elongate object as in claim 1.

A device constructed according to the exemplary embodiment further includes various additional features. For example, a valve comprised of a valve member and a valve seat selectively supplies the coating material to the elongate chamber. A ring shaped structure communicates between the air supply passage and the mixing passage. The ring shaped structure is configured to cause the pressurized air to enter the mixing passage with a swirling motion about a discharge location of the coating material into the mixing passage. The ring shaped further includes a central passage through which the coating material is directed into the mixing passage and into the air moving with the swirling motion. The device may further include a coating material injecting element including a tube with an outlet. The tube extends through the central passage with the outlet of the tube positioned in the mixing passage. The ring shaped structure more specifically includes a ring shaped wall surrounding an inner space and disposed around a central axis. The central passage extends along the central axis. The ring shaped wall further includes a plurality of air directing passages communicating between the air supply passage and the inner space so as to achieve the swirling motion around the central axis. The ring shaped structure further comprises a plurality of stand-off elements forming additional air passages between the stand-off elements and providing communication between the air supply passage and the mixing passage.

The elongate chamber is preferably configured with increasing diameter in a direction from the first end to the second end so that an increase in pressure is achieved in this direction within the chamber. The flow of the mist is generally along a central axis of the elongate chamber coaxial with the port at the first end. The mixing passage extends transverse, and more preferably perpendicular, to the elongate chamber.

The elongate object further comprises a hollow needle having opposite, open ends, such as a hypodermic syringe needle. The device further comprises a needle holder including an interior air space and a needle holding element configured to secure a first end of the needle in communication with the interior air space. The open second end of the needle extends outwardly from the needle holder for insertion through the port and into the elongate chamber. The interior air space is adapted to be pressurized with air to force air through the needle during a coating operation and prevent clogging of the open second end of the needle with the coating material.

In another aspect, a method is provided for coating an exterior surface of an elongate object with a coating material as described in claim 5. The method generally includes holding the elongate object lengthwise in an elongate chamber having first and second opposite ends and an outlet at the second end. A mist is formed from a mixture of the air and the coating material and the exterior surface is coated with the coating material while directing the mist around the exterior surface of the elongate object and toward the outlet of the elongate chamber.

The method practiced according to an illustrative example includes various additional aspects and steps. For example, the pressurized air and the coating material are first mixed within a mixing passage oriented transverse to the elongate chamber prior to directing the air and the coating material into the elongate chamber as a mist. The mixing passage extends along an axis and the method further comprises swirling the air around the axis of the mixing passage at an air inlet to the mixing passage, and directing the coating material into the mixing passage along the central axis to mix with the swirling air. The method further comprises directing the air into the mixing passage with a Venturi effect. Holding the elongate object further comprises directing the object through a port at the first end of the elongate chamber and the method further comprises directing additional air through the port and adjacent the object toward the outlet of the elongate chamber. The elongate object further comprises a hollow needle with opposite, first and second open ends, and the method further comprises directing air through the first open end of the needle and out of the second open end positioned in the elongate chamber to prevent clogging of the first open end with the coating material. Directing the coating material into the elongate chamber further comprises actuating a valve to allow pressurized coating material to flow into the elongate chamber. The coating material may be a friction reducing material, such as a material containing silicone.

Various additional features and advantages of the invention will become more apparent to those of ordinary skill in the art upon review of the following detailed description of the illustrative embodiments taken in conjunction with the accompanying drawings.

### Brief Description of the Drawings

Fig. 1 is a perspective view of a device constructed in accordance with an illustrative embodiment of the invention.
Fig. 2 is a cross sectional view generally taken along line 2-2 of Fig. 1 and rotated 90° for purposes of easier explanation.
Fig. 2A is an enlarged view of the portion marked "2A" in Fig. 2.
Fig. 3 is a perspective view of the ring shaped structure used to direct the air flow within the device of Fig. 1.
Fig. 4 is a cross sectional view taken generally along line 4-4 of Fig. 3.

### Detailed Description of the Illustrative Embodiments

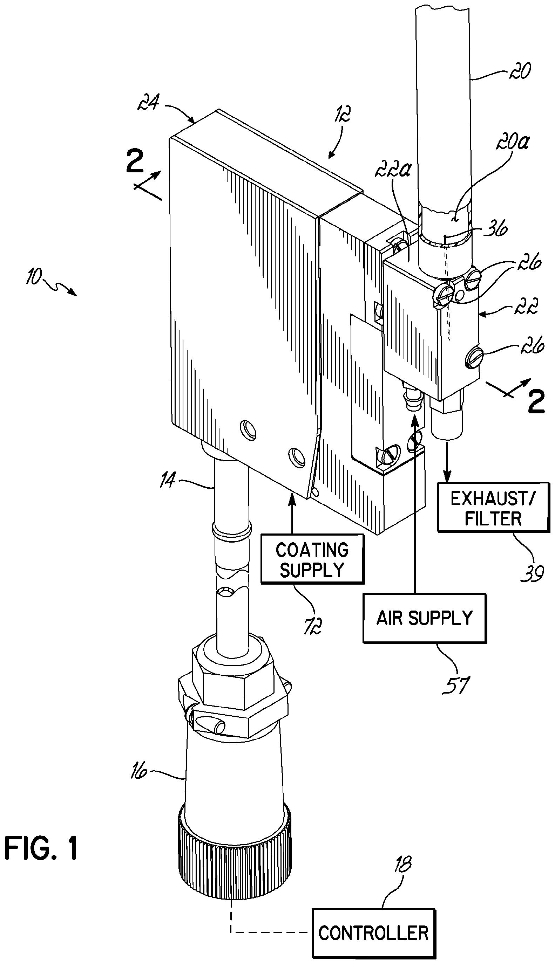

Fig. 1 illustrates a device 10 generally comprising a housing structure 12 coupled with a conduit 14 suitable for containing control and electrical supply wiring (not shown). An electrical coupler 16 is provided and adapted to couple the device 10 to a controller 18 for operating the device 10. A needle holder 20 is provided for introducing a needle to be coated, as further discussed below, into the housing structure 12. More specifically, the needle holder 20 is adapted to abut an outside face 22a of a first sub-housing 22 coupled to a second sub-housing 24 of the housing structure 12 by fasteners 26.

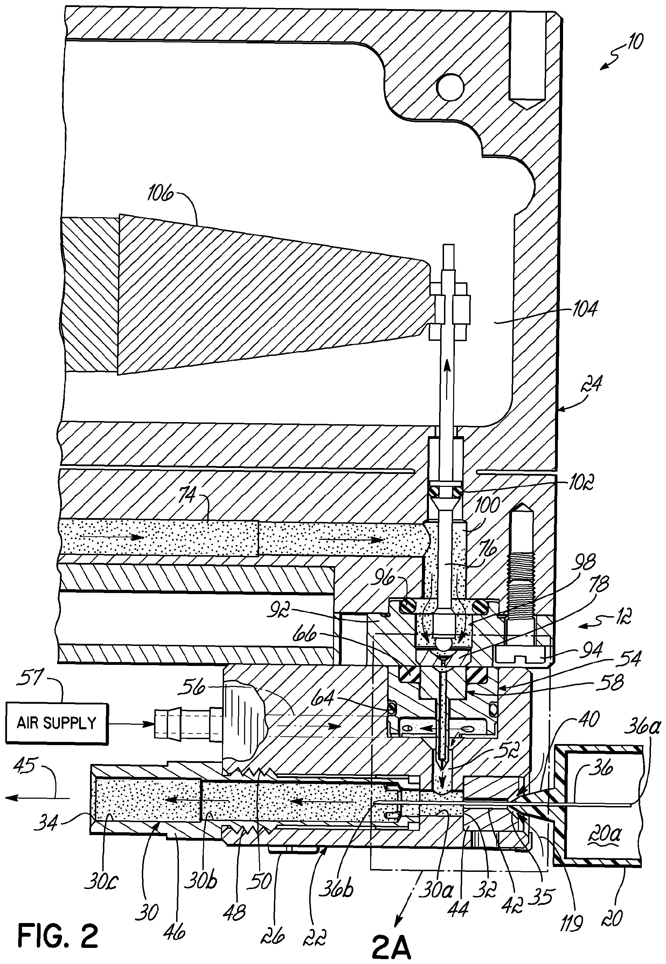

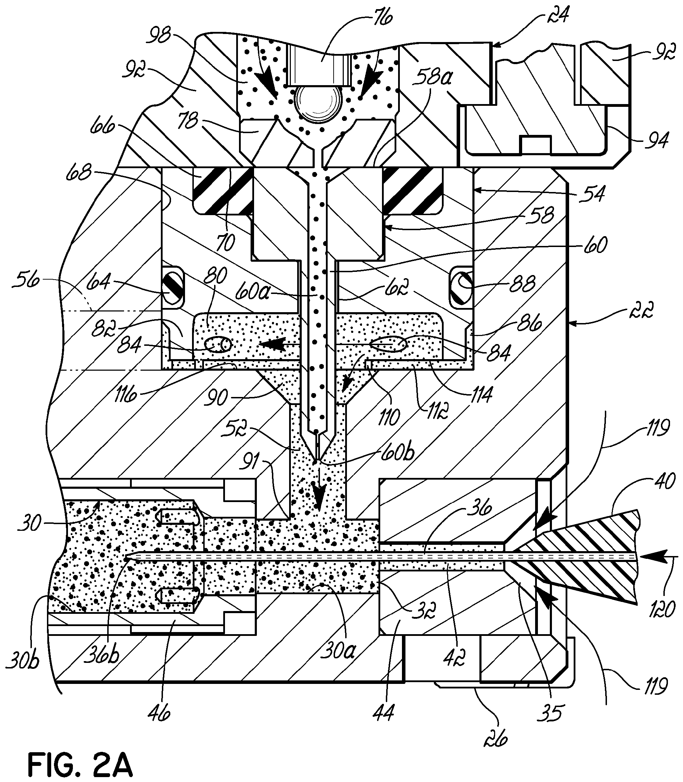

Referring to Figs. 2 and 2A, the first sub-housing 22 more specifically comprises an elongate chamber 30 having first and second opposite ends 32, 34. A port 35 at the first end 32 receives a needle 36 for coating purposes. The second end 34 defines an outlet. As shown in Fig. 1, the second end is fluidly coupled to an exhaust 39 including a filter. The needle holder 20 is mounted for movement in a manner (not shown) that allows for repeatable introduction of successive needles 36 into the chamber 30 during a manufacturing operation. The needle holder 20 includes an interior air space 20a and a needle holding element 40. The needle holding element 40 grips the needle 36 with friction or in any other suitable manner such that an open first end 36a communicates with the interior air space 20a and an open second end 36b is positionable within the elongate chamber 30. The needle 36 extends through the port 35 preferably along the central axis of the port 35 and the elongate chamber 30. The needle 36 extends through a passage 42 in an insert 44 within the first sub-housing 22 and the port 35 more specifically comprises a conically shaped opening in the insert 44. As will be more specifically described below, air is drawn in through the conically shaped port 35 into the passage 42 of the insert 44 and, finally, into the elongate chamber 30. The elongate chamber 30 comprises cylindrical chamber portions 30a, 30b, 30c of successively larger diameter for creating an increasing pressure as pressurized air flows from the first end 32 toward the second end 34 in the direction of the arrows 45. At least a portion of the elongate chamber 30 may be formed by the interior of a tubular member 46 that is removably attached to the first sub-housing 22 by respective threads 48, 50.

A mixing passage 52 in the first sub-housing 22 extends transverse and, more specifically, perpendicular to the elongate chamber 30. This mixing passage 52 communicates directly with the smallest diameter chamber portion 30a adjacent the insert 44 and needle port 35. As best shown in Fig. 2A, the first sub-housing 22 further includes a ring shaped structure 54 for receiving pressurized air from an air supply passage 56. The air supply passage 56 is coupled with a source 57 of pressurized air. The pressure of the air is preferably in the range of one to four bar. The ring shaped structure 54 further receives a coating material injecting element 58 including a tube 60 with a passage 60a and an outlet 60b extending through a central passage 62 of the ring shaped structure 54. The ring shaped structure 54 more specifically comprises a ring sealed by respective O-rings 64, 66 within a recess 68 of the first sub-housing 22 and against a surface 70 of the second sub-housing 24.

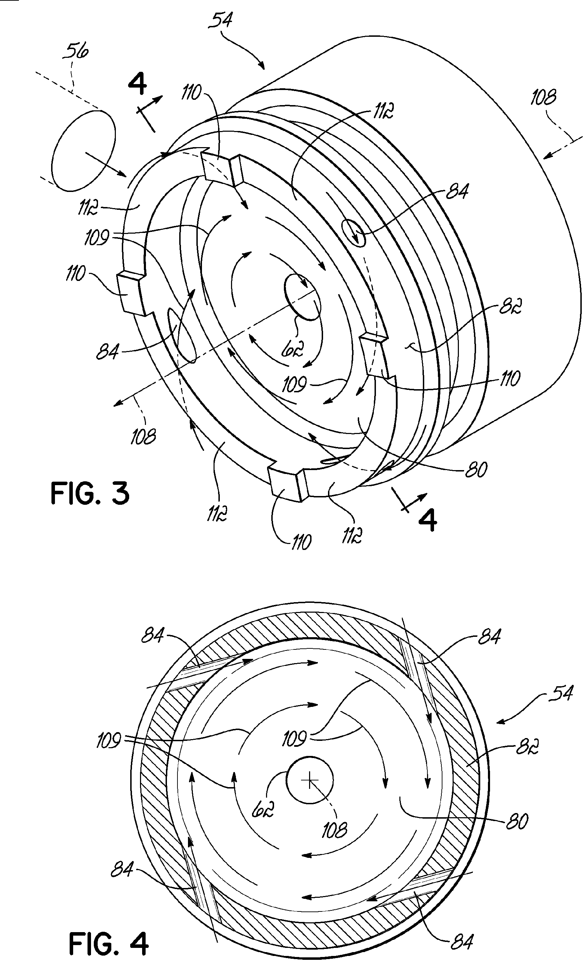

The coating material is provided from a suitable pressurized supply 72 (Fig. 1). In the case of coating a hypodermic syringe needle, for example, the coating materials are preferably silicone oils of various viscosity ranging, for example, from the viscosity of water to 12,500 mPas. Most applications currently contemplated will utilize a viscosity of 1000 - 3000 mPas. The coating material is directed through a supply passage 74 (Fig. 2) and ultimately into the central passage 60a of the tube 60 and is injected into the mixing passage 52 when a valve element 76 positioned in the second sub-housing 24 is opened relative to a valve seat element 78 abutting an inlet end 58a of the coating material injecting element 58. As will be described further in connection with Figs. 3 and 4, the ring shaped structure 54 receives and directs the air in a swirling motion within an inner space 80 defined by a ring shaped or annular wall 82. The ring shaped wall 82 includes a plurality of air directing passages 84 communicating with the air supply passage 56 through an annular air space 86 formed between the ring shaped wall 82 and the wall 88 of the recess 68. The swirling air within an inner space 80 is directed downwardly into a generally cone-shaped or converging passage 90 surrounding the tube 60 of the coating material injecting element 58 and then into the mixing passage 52. Due to the constriction formed by the converging passage 90, the air is directed into the mixing passage with a Venturi effect in addition to the swirling motion. The air then mixes with the injected coating material to form a mixture in the form of a mist as the air and coating material enter the elongate chamber 30 through a connecting port 91.

More specifically referring to Fig. 2, the valve seat element 78 is carried on a valve component 92 affixed to a main part of the second sub-housing 24 by threaded fasteners 94 (only one shown). The valve component 92 is mounted in sealing engagement with the main part of the second sub-housing 24 through the use of an O-ring 96. A passage 98 of the valve component 92 communicates with a passage 100 in the second sub-housing 24 containing the reciprocating valve element or valve stem 76. The passage 100 communicates with the coating material supply passage 74 and, ultimately, with the coating material supply 72 (Fig. 1). The valve stem 76 includes an additional dynamic seal 102 engaged with the internal wall defining the passage 100 to prevent the coating material from leaking into the interior space 104 of the second sub-housing 24 which contains valve stem actuating components, including an actuating arm 106. The valve stem 76 and actuating arm 106 may be actuated in any suitable manner such as by using a conventional piezoelectric actuator (not shown).

Referring to Fig. 2A in combination with Figs. 3 and 4, the ring shaped structure 54 includes the ring shaped wall 82 surrounding the inner space 80 and the wall 82 is spaced around a central axis 108. The central passage 62 extends in a coaxial fashion around the central axis 108 and the ring shaped wall 82 further includes the plurality of air directing passages 84 communicating with the annular space 86 (Fig. 2A) which, in turn, communicates with the pressurized air supply passage 56. The air directing passages 84 are formed in a generally tangential manner relative to the cylindrical shape of the inner space 80 as best illustrated in Fig. 4 such that air entering the inner space 80 travels with a swirling motion as shown by the arrows 109. A plurality of stand-off elements 110 extends from a lower end of the ring shaped structure 54 and provide for additional air passages or paths 112 (Fig. 2A) between the stand-off elements 110 and between lower surfaces 114 of the ring shaped structure 54 and the bottom wall 116 (Fig. 2A) of the recess 68, as shown in Fig. 2A. Therefore, air will enter an annular space 86 from the air supply passage 56 and, from the annular space 86, pass through the air directing passages 84 into the inner space 80, and also pass between the stand-off elements 110 through passages 112. The air passing through the air directing passages 84 will cause a swirling air motion within the inner space 80 and this air will be directed downwardly to combine with the air passing between the stand-off elements 110 and then enter the inlet 90 of the air mixing passage 52 with a swirling motion and Venturi effect, i.e., reduced pressure and increased velocity. This mixing action will begin to form a mist with the coating material being injected through the outlet 60b of the tube 60 along the central axis 108 of the ring shaped structure 54 and the coaxial central axis of the mixing passage 52. The flow within the elongate chamber 30 will draw additional air into the port 35 as shown by arrows 119 and through the passage 42 of the insert 44 (see Figs. 2 and 2A) to prevent any coating material from traveling in the opposite direction through the port 35. At the same time, pressurized air in the interior air space 20a will travel as illustrated by the arrow 120 in Fig. 2A through the open first end 36a of the needle 36 and out of the open second end 36b of the needle 36 to prevent the second end 36b of the needle 36 from being clogged by coating material in the mist. The controller 18 (Fig. 1) will operate the actuating arm 106 to open and close the valve stem 76, and operate the pressurized air in an on/off fashion as necessary to successively coat multiple needles 36 in serial fashion as they are introduced one-by-one into the elongate chamber 30 by the same or different needle holders 20.

The first sub-housing 22 may be removed from the second sub-housing 24 for repair and replacement purposes. More specifically, components contained in the first sub-housing 22 may become contaminated due to the environmental air that is being suctioned into the passages of those components and mixed with the coating material. On the other hand, the components and passages associated with the second sub-housing 24 need much less service during regular use. Thus, the second sub-housing 24 and its associated components may remain mounted while the first sub-housing 22 and its attached components are removed from the second sub-housing by removing fasteners 26. The components 44, 46, 54, 58 associated with the first sub-housing 22 may be easily disassembled for cleaning purposes. In addition, the modular nature of the first sub-housing 22 allows different application requirements to be accommodated, such as size changes to accommodate different elongate objects, and adjustments to the type and/or viscosity of coating material. In addition, the injecting element 58 may be replaced to accommodate a coating material having a different viscosity. Finally, the insert 44 receiving the elongate object 36 may be modified for particular applications, such as coating operations of syringe tips with various configurations.

While the present invention has been illustrated by a description of various preferred embodiments and while these embodiments have been described in some detail, it is not the intention of the Applicants to restrict or in any way limit the scope of the appended claims to such detail. Additional advantages and modifications will readily appear to those skilled in the art. The various features of the invention may be used alone or in any combination depending on the needs and preferences of the user. This has been a description of the present invention, along with the preferred methods of practicing the present invention as currently known. However, the invention itself should only be defined by the appended claims.

A device for coating an exterior of an elongate object includes a housing structure with an elongate chamber having first and second opposite ends. A port communicates with the first end for receiving the elongate object and an outlet is located at the second end. An air supply passage and a coating material supply passage communicate with the elongate chamber. Pressurized air and coating material are adapted to enter the elongate chamber through the air supply passage and the coating material supply passage, respectively, to form a mist in the elongate chamber moving toward the outlet while coating the exterior of the object. A method of coating an exterior surface of the elongate object with the coating material includes holding the elongate object lengthwise in the elongate chamber, mixing the pressurized air and the coating material to form a mist, and coating the exterior surface while directing the mist around the exterior surface and toward the outlet of the elongate chamber.

The invention is further described by the following embodiments, wherein:
Embodiment 1. A device for coating an exterior of an elongate object, comprising:
   a housing structure including an elongate coating chamber having first and second opposite ends, a port communicating with said first end for receiving the elongate object into the coating chamber and an outlet at said second end, said coating chamber having at least first and second sections, said first section located closer to said outlet than said second section and said first section having a greater cross sectional area than said second section, the housing structure further including an air supply passage and a coating material supply passage communicating with said elongate coating chamber;
   wherein pressurized air and coating material are adapted to enter said elongate chamber through said air supply passage and said coating material supply passage, respectively, to form a mist of the air and coating material that is directed into said elongate chamber and generally toward said outlet while coating the exterior of the elongate object inserted into said elongate chamber through said port.
Embodiment 2. The device with the features of embodiment 1, further comprising:
   a mixing passage communicating with said air supply passage and said coating material supply passage, said mixing passage further communicating with said elongate chamber, wherein pressurized air and coating material are adapted to enter said mixing passage through said air supply passage and said coating material supply passage, respectively, to form the mist of the air and coating material that is directed into said elongate chamber and generally toward said outlet while coating the exterior of the object inserted into said elongate chamber through said port.
Embodiment 3. The device with the features of embodiment 2, wherein said elongate chamber includes a central axis coaxial with said port, and said mixing passage extends transverse to said elongate chamber.
Embodiment 4. The device with the features of embodiment 1, further comprising:
   a ring shaped structure communicating between said air supply passage and said elongate chamber, said ring shaped structure configured to cause a swirling motion of the pressurized air, said ring shaped structure further including a central passage through which the coating material is adapted to be directed into the air moving with the swirling motion to thereby form the mist.
Embodiment 5. The device with the features of embodiment 4, further comprising a coating material injecting element including a tube with an outlet, said tube extending through said central passage.
Embodiment 6. The device with the features of embodiment 5, wherein said ring shaped structure further comprises a ring shaped wall surrounding an inner space and disposed around a central axis, and wherein said central passage extends along the central axis, said ring shaped wall further including a plurality of air directing passages communicating between said air supply passage and said inner space and directing the pressurized air into said inner space in the swirling motion around said central axis.
Embodiment 7. The device with the features of embodiment 4, wherein said ring shaped structure further comprises a plurality of stand-off elements forming additional air passages communicating between said air supply passage and said elongate chamber.
Embodiment 8. The device with the features of embodiment 1, further comprising:
   a valve member and a valve seat mounted in said coating material supply passage, said valve member movable with respect to said valve seat to selectively supply the coating material to the elongate chamber.
Embodiment 9. The device with the features of embodiment 8, wherein said housing structure further comprises a first sub-housing and a second sub-housing, said first sub-housing being removably coupled to said second sub-housing and containing said elongate coating chamber, and said second sub-housing containing said valve member and said valve seat.
Embodiment 10. The device with the features of embodiment 1, wherein the elongate object further comprises a hollow needle, and further comprising:
   a needle holder including an interior air space and a needle holding element configured to secure a first end of the needle in communication with the interior air space while an open second end of the needle extends outwardly from said needle holder for insertion through said port and into said elongate chamber, wherein said interior air space is adapted to be pressurized with air to force air through the needle and prevent clogging of the open second end of the needle with the coating material.
Embodiment 11. The device with the features of embodiment 1, wherein said port is configured to allow additional air to be drawn into said elongate chamber as the mist is coating the exterior of the elongate object.
Embodiment 12. A device for coating an exterior of an elongate object, comprising:
   a housing structure including an elongate coating chamber having first and second opposite ends, said elongate coating chamber including a port communicating with said first end for receiving the elongate object and an outlet at said second end, an air supply passage, a coating material supply passage, and a mixing passage communicating with said air supply passage and said coating material supply passage, said mixing passage further communicating with said elongate coating chamber; and
   a ring shaped structure communicating between said air supply passage and said mixing passage, said ring shaped structure configured to cause the pressurized air to enter said mixing passage with a swirling motion, said ring shaped structure further including a central passage through which the coating material is directed into said mixing passage and into the air moving with the swirling motion;
   wherein the pressurized air and coating material in said mixing passage form a mist in said elongate chamber moving toward said outlet while coating the exterior of an object inserted into said elongate chamber through said port
Embodiment 13. The device with the features of embodiment 12, wherein said ring shaped structure further comprises a ring shaped wall surrounding an inner space and disposed around a central axis, and wherein said central passage extends along the central axis, said ring shaped wall further including a plurality of air directing passages communicating between said air supply passage and said inner space and directing the pressurized air into said inner space in the swirling motion around the central axis.
Embodiment 14. The device with the features of embodiment 13, wherein said ring shaped wall further comprises a plurality of stand-off elements forming additional air passages communicating between said air supply passage and said mixing passage.
Embodiment 15. The device with the features of embodiment 12, further comprising a coating material injecting element including a tube with an outlet, said tube extending through said central passage with said outlet positioned in said mixing passage.
Embodiment 16. The device with the features of embodiment 12, further comprising:
   a valve member and a valve seat mounted in said coating material supply passage, said valve member movable with respect to said valve seat to selectively supply the coating material to the elongate chamber.
Embodiment 17. The device with the features of embodiment 12, wherein said port is configured to allow additional air to be drawn into said elongate chamber as the mist is coating the exterior of the elongate object. Embodiment 18. The device with the features of embodiment 12, wherein said elongate chamber includes a central axis coaxial with said port, and said mixing passage extends transverse to said elongate chamber.
Embodiment 19. The device with the features of embodiment 12, wherein the elongate object further comprises a hollow needle, and further comprising:
   a needle holder including an interior air space and a needle holding element configured to secure a first end of the needle in communication with the interior air space while an open second end of the needle extends outwardly from said needle holder for insertion through said port and into said elongate chamber, wherein said interior air space is adapted to be pressurized with air to force air through the needle and prevent clogging of the open second end of the needle with the coating material.
Embodiment 20. The device with the features of embodiment 12, wherein said ring shaped structure includes an air passage forming a constriction into said mixing passage thereby causing a Venturi effect of the air entering said mixing passage.
Embodiment 21. The device with the features of embodiment 12, further comprising:
   a valve member and a valve seat mounted in said coating material supply passage, said valve member movable with respect to said valve seat to selectively supply the coating material to the elongate chamber.
Embodiment 22. The device with the features of embodiment 21, wherein said housing structure further comprises a first sub-housing and a second sub-housing, said first sub-housing being removably coupled to said second sub-housing and containing said mixing passage, said ring shaped structure and said elongate coating chamber, and said second sub-housing containing said valve member and said valve seat.
Embodiment 23. The device with the features of embodiment 12, wherein said port is configured to allow additional air to be drawn into said elongate chamber as the mist is coating the exterior of the elongate object.
Embodiment 24. A method of coating an exterior surface of an elongate object with a coating material, comprising:
   holding the elongate object lengthwise in an elongate chamber having first and second opposite ends and an outlet at the second end,
   mixing pressurized air and the coating material to form a mist; and
   coating the exterior surface with the coating material while directing the mist around the exterior surface and toward the outlet of the elongate chamber.
Embodiment 25. The method with the features of embodiment 24, further comprising:
   mixing the pressurized air and the coating material in a mixing passage oriented transverse to the elongate chamber prior to directing the air and the coating material into the elongate chamber.
Embodiment 26. The method with the features of embodiment 25, wherein the mixing passage extends along an axis, and the method further comprises:
   swirling the air around the axis at an air inlet to the mixing passage; and
   directing the coating material into the mixing passage along the central axis to mix with the swirling air.
Embodiment 27. The method with the features of embodiment 26, further comprising:
   directing the air into the mixing passage with a Venturi effect.
Embodiment 28. The method with the features of embodiment 24, wherein holding the elongate object further comprises directing the object through a port communicating with the first end of the elongate chamber, and the method further comprises:
   directing additional air through the port and adjacent the object toward the outlet of the elongate chamber.
Embodiment 29. The method with the features of embodiment 24, wherein the elongate object further comprises a hollow needle with opposite, first and second open ends, and the method further comprises:
   locating the second open end in the elongate chamber; and
   directing air through the first open end of the needle and out of the second open end to prevent clogging of the second open end with the coating material.
Embodiment 30. The method with the features of embodiment 24, wherein directing the coating material further comprises:
   actuating a valve to allow pressurized coating material to flow into the elongate chamber.
Embodiment 31. The method with the features of embodiment 24, wherein the coating material comprises a friction reducing material.
Embodiment 32. The method with the features of embodiment 24, wherein directing the mist toward the outlet further comprises:
   directing the mist through sections of the chamber having increasing diameter.

## Claims

1. A device (10) for coating an exterior of an elongate object, comprising:
a housing structure (12) including an elongate coating chamber (30) having first and second opposite ends (32, 34), said elongate coating chamber including a port (35) communicating with said first end (32) for receiving the elongate object and an outlet at said second end (34), an air supply passage (56), a coating material supply passage (74), and a mixing passage (52) communicating with said air supply passage (56) and said coating material supply passage (74), said mixing passage (52) further communicating with said elongate coating chamber (30); and
a ring shaped structure (54) communicating between said air supply passage (56) and said mixing passage (52), said ring shaped structure (54) configured to cause the pressurized air to enter said mixing passage (52) with a swirling motion, said ring shaped structure (54) further including a central passage (62) through which the coating material is directed into said mixing passage (52) and into the air moving with the swirling motion;
wherein the pressurized air and coating material in said mixing passage (52) form a mist in said elongate chamber (30) moving toward said outlet while coating the exterior of an object inserted into said elongate chamber (30) through said port (35).

2. The device of claim 1, wherein said ring shaped structure (54) further comprises a ring shaped wall (82) surrounding an inner space (80) and disposed around a central axis, and wherein said central passage (62) extends along the central axis (108), said ring shaped wall (82) further including a plurality of air directing passages communicating between said air supply passage and said inner space (80) and directing the pressurized air into said inner space (80) in the swirling motion around the central axis (108), and/or
wherein said ring shaped wall (82) further comprises a plurality of stand-off elements (110) forming additional air passages communicating between said air supply passage (56) and said mixing passage(52).

3. The device of claim 1, further comprising a coating material injecting element (58) including a tube (60) with an outlet (60b), said tube (60) extending through said central passage (62) with said outlet positioned in said mixing passage (52); and/or further comprising:
a valve member (76) and a valve seat (78) mounted in said coating material supply passage (74), said valve member (76) movable with respect to said valve seat (78) to selectively supply the coating material to the elongate chamber (30); and/or
wherein said port (35) is configured to allow additional air to be drawn into said elongate chamber (30) as the mist is coating the exterior of the elongate object; and/or
wherein said elongate chamber (30) includes a central axis (108) coaxial with said port (35), and said mixing passage extends transverse to said elongate chamber; and/or
wherein the elongate object further comprises a hollow needle, and further comprising:
a needle holder (20) including an interior air space (20a) and a needle holding element (40) configured to secure a first end (36a) of the needle (36) in communication with the interior air space (20a) while an open second end (36b) of the needle extends outwardly from said needle holder (20) for insertion through said port (35) and into said elongate chamber (30), wherein said interior air space (20a) is adapted to be pressurized with air to force air through the needle (36) and prevent clogging of the open second end (36b) of the needle (36) with the coating material; and/or
wherein said ring shaped structure (54) includes an air passage forming a constriction into said mixing passage (52) thereby causing a Venturi effect of the air entering said mixing passage (52); and/or
further comprising:
a valve member (76) and a valve seat (78) mounted in said coating material supply passage (74), said valve member (76) movable with respect to said valve seat (78) to selectively supply the coating material to the elongate chamber (30); and/or
wherein said (35) port is configured to allow additional air to be drawn into said elongate chamber (30) as the mist is coating the exterior of the elongate object.

4. The device of claim 3, wherein said housing structure (12) further comprises a first sub-housing (2) and a second sub-housing (24), said first sub-housing (22) being removably coupled to said second sub-housing (24) and containing said mixing passage (52), said ring shaped structure (54) and said elongate coating chamber (30), and said second sub-housing (24) containing said valve member (76) and said valve seat (78).

5. A method of coating an exterior surface of an elongate object with a coating material under use of a device (10) according to claim 1, comprising:
holding the elongate object lengthwise in an elongate chamber (30) having first and second opposite ends (32, 34) and an outlet at the second end,
mixing pressurized air and the coating material to form a mist; and coating the exterior surface with the coating material while directing the mist around the exterior surface and toward the outlet of the elongate chamber (30).

6. The method of claim 5, further comprising:
mixing the pressurized air and the coating material in a mixing passage (52) oriented transverse to the elongate chamber (30) prior to directing the air and the coating material into the elongate chamber (30),
wherein preferably the mixing passage (30) extends along an axis (108), and the method further comprises:
swirling the air around the axis (108) at an air inlet to the mixing passage (52); and
directing the coating material into the mixing passage (52) along the central axis (108) to mix with the swirling air.

7. The method of claim 6, further comprising:
directing the air into the mixing passage (52) with a Venturi effect.

## Patentansprüche

1. Eine Vorrichtung (10) zum Beschichten der Außenseite eines länglichen Objektes, aufweisend:
eine Gehäusestruktur (12) mit einer langgestreckten Beschichtungskammer (30) mit ersten und zweiten, gegenüberliegenden Enden (32, 34), wobei die langgestreckte Beschichtungskammer eine Öffnung (35) umfasst, welche mit dem ersten Ende (32) zum Aufnehmen des länglichen Objektes kommuniziert, und einen Auslass an dem zweiten Ende (34) umfasst, einen Luftzuführkanal (56), einen Beschichtungsmaterial-Versorgungsdurchgang (74) und einen Mischdurchgang (52), der mit dem Luftzuführkanal (56) und dem Beschichtungsmaterial-Versorgungsdurchgang (74) in Verbindung steht, wobei der Mischdurchgang (52) ferner mit der langgestreckten Beschichtungskammer (30) in Verbindung steht; und
eine ringförmige Struktur (54), welche zwischen dem Luftzuführkanal (56) und dem Mischdurchgang (52) in Verbindung steht, wobei die ringförmige Struktur (54) dazu eingerichtet ist, dass die Druckluft in den Mischdurchgang (52) mit einer Verwirbelungsbewegung eintritt, wobei die ringförmige Struktur (54) ferner einen zentralen Durchgang (62) umfasst, durch welchen das Beschichtungsmaterial in den Mischdurchgang (52) und in die Luft, die sich mit der Verwirbelungsbewegung bewegt, gerichtet ist;
wobei die Druckluft und das Beschichtungsmaterial in dem Mischdurchgang (52) einen Nebel in der langgestreckten Kammer (30) ausbilden, der sich in Richtung des Auslasses bewegt, während die Außenseite eines in die langgestreckte Kammer (30) eingeführten Objektes durch die Öffnung (35) beschichtet wird.

2. Die Vorrichtung nach Anspruch 1, wobei die ringförmige Struktur (54) ferner eine ringförmige Wand (82) aufweist, die einen Innenraum (80) umgibt und um eine Mittenachse angeordnet ist, und wobei der zentrale Durchgang (62) sich entlang der Mittenachse (108) erstreckt, wobei die ringförmige Wand (82) ferner eine Vielzahl von Luftleitkanälen umfasst, welche zwischen dem Luftzuführkanal und dem Innenraum (80) in Verbindung stehen und die Druckluft in den Innenraum (80) in die Verwirbelungsbewegung um die Mittenachse (108) herum leiten, und/oder
wobei die ringförmige Wand (82) ferner eine Vielzahl Abstandselemente (110) aufweist, welche zusätzliche Luftdurchgänge ausbilden, welche zwischen dem Luftzuführkanal (56) und dem Mischdurchgang (52) in Verbindung stehen.

3. Die Vorrichtung nach Anspruch 1, ferner aufweisend ein Beschichtungsmaterial-Einspritzelement (58) umfassend ein Rohr (60) mit einem Auslass (60b), wobei das Rohr (60) sich durch den zentralen Durchgang (62) erstreckt, wobei der Auslass in dem Mischdurchgang (52) angeordnet ist; und/oder
ferner aufweisend:
ein Ventilelement (76) und einen Ventilsitz (78), die in dem Beschichtungsmaterial-Versorgungsdurchgang (74) montiert sind, wobei das Ventilelement (76) in Bezug auf den Ventilsitz (78) bewegbar ist, um wahlweise das Beschichtungsmaterial der langgestreckten Kammer (30) zuzuführen; und/oder
wobei die Öffnung (35) dazu eingerichtet ist zu ermöglichen, dass zusätzliche Luft in die langgestreckte Kammer (30) gesogen wird, wenn der Nebel die Außenseite des länglichen Objektes beschichtet; und/oder
wobei die langgestreckte Kammer (30) eine Mittenachse (108) umfasst, welche koaxial mit der Öffnung (35) ist und wobei der Mischdurchgang sich quer zu der länglichen Kammer erstreckt; und/oder
wobei das längliche Objekt ferner eine hohle Nadel aufweist, und ferner aufweisend:
einen Nadelhalter (20), umfassend einen inneren Luftraum (20a) und ein Nadel-Halteelement (40), das dazu eingerichtet ist, das erste Ende (36a) der Nadel (36) zu befestigen, das in Verbindung mit dem inneren Luftraum (20a) steht, während sich ein offenes zweites Ende (36b) der Nadel nach außen von dem Nadelhalter (20) zum Einführen durch die Öffnung (35) und in die langgestreckte Kammer (30) erstreckt, wobei der innere Luftraum (20a) dazu eingerichtet ist, unter Druckluft gesetzt zu werden, um Luft durch die Nadel (36) zu drücken und ein Verstopfen des offenen zweiten Endes (36b) der Nadel (36) mit dem Beschichtungsmaterial zu verhindern; und/oder wobei die ringförmige Struktur (54) einen Luftdurchgang umfasst, der eine Einschnürung in dem Mischdurchgang (52) ausbildet, wodurch ein Venturi-Effekt der in den Mischdurchgang (52) eintretenden Luft bewirkt ist; und/oder
ferner aufweisend:
ein Ventilelement (76) und einen Ventilsitz (78), die in dem Beschichtungsmaterial-Versorgungsdurchgang (74) montiert sind, wobei das Ventilelement (76) in Bezug auf den Ventilsitz (78) bewegbar ist, um wahlweise das Beschichtungsmaterial der langgestreckten Kammer (30) zuzuführen; und/oder
wobei die Öffnung (35) dazu eingerichtet ist zu ermöglichen, dass zusätzlich Luft in die langgestreckte Kammer (30) gesogen wird, wenn der Nebel die Außenseite des länglichen Objektes beschichtet.

4. Die Vorrichtung nach Anspruch 3, wobei die Gehäusestruktur (12) ferner ein erstes Untergehäuse (2) und ein zweites Untergehäuse (24) aufweist, wobei das erste Untergehäuse (22) entfernbar mit dem zweiten Untergehäuse (24) gekoppelt ist und den Mischdurchgang (52) enthält, wobei die ringförmige Struktur (54) und die langgestreckte Beschichtungskammer (30) und das zweite Untergehäuse (24) das Ventilelement (76) und den Ventilsitz (78) enthalten.

5. Ein Verfahren zum Beschichten einer äußeren Oberfläche eines länglichen Objektes mit einem Beschichtungsmaterial unter Verwendung der Vorrichtung (10) gemäß Anspruch 1, aufweisend:
Halten des länglichen Objektes in Längsrichtung in einer langgestreckten Kammer (30) mit ersten und zweiten, gegenüberliegenden Enden (32, 34) und einem Auslass an dem zweiten Ende,
Mischen von Druckluft und dem Beschichtungsmaterial, um einen Nebel zu bilden; und Beschichten der äußeren Oberfläche mit dem Beschichtungsmaterial, während der Nebel um die äußere Oberfläche herum und in Richtung des Auslasses der langgestreckten Kammer (30) geleitet wird.

6. Das Verfahren nach Anspruch 5, ferner aufweisend:
Mischen der Druckluft und des Beschichtungsmaterials in einem Mischdurchgang (52), der quer zu der langgestreckten Kammer (30) orientiert ist, vor dem Einleiten der Luft und dem Beschichtungsmaterial in die langgestreckte Kammer (30),
wobei vorzugsweise der Mischdurchgang (30) sich entlang einer Achse (108) erstreckt, und das Verfahren ferner aufweist:
Verwirbeln der Luft um die Achse (108) an einem Lufteinlass zu dem Mischdurchgang (52); und
Einleiten des Beschichtungsmaterials in den Mischdurchgang (52) entlang der Mittenachse (108), um dieses mit der wirbelnden Luft zu vermischen.

7. Das Verfahren nach Anspruch 6, ferner aufweisend:
Einleiten der Luft in den Mischdurchgang (52) mit einem Venturi-Effekt.

## Revendications

1. Dispositif (10) de revêtement d'un extérieur d'un objet allongé, comprenant :
une structure de logement (12) comportant une chambre de revêtement allongée (30) ayant des première et deuxième extrémités opposées (32, 34), ladite chambre de revêtement allongée comportant un orifice (35) communiquant avec ladite première extrémité (32) pour recevoir l'objet allongé et une sortie à ladite deuxième extrémité (34), un passage de fourniture d'air (56), un passage de fourniture de matériau de revêtement (74) et un passage de mélange (52) communiquant avec ledit passage de fourniture d'air (56) et ledit passage de fourniture de matériau de revêtement (74), ledit passage de mélange (52) communiquant en outre avec ladite chambre de revêtement allongée (30) ; et
une structure de forme annulaire (54) communiquant entre ledit passage de fourniture d'air (56) et ledit passage de mélange (52), ladite structure de forme annulaire (54) étant configurée pour entraîner l'entrée de l'air pressurisé dans ledit passage de mélange (52) avec un mouvement tourbillonnant, ladite structure de forme annulaire (54) comportant en outre un passage central (62) au travers duquel le matériau de revêtement est dirigé dans ledit passage de mélange (52) et dans l'air se déplaçant avec le mouvement tourbillonnant ;
dans lequel l'air pressurisé et le matériau de revêtement dans ledit passage de mélange (52) forment une brume dans ladite chambre allongée (30) se déplaçant vers ladite sortie tout en revêtant l'extérieur d'un objet inséré dans ladite chambre allongée (30) par ledit orifice (35).

2. Dispositif selon la revendication 1, dans lequel ladite structure de forme annulaire (54) comprend en outre une paroi de forme annulaire (82) entourant un espace intérieur (80) et disposée autour d'un axe central, et dans lequel ledit passage central (62) s'étend le long de l'axe central (108), ladite paroi de forme annulaire (82) comportant en outre une pluralité de passages de direction d'air communiquant entre ledit passage de fourniture d'air et ledit espace intérieur (80) et dirigeant l'air pressurisé dans ledit espace intérieur (80) dans le mouvement tourbillonnant autour de l'axe central (108), et/ou
dans lequel ladite paroi de forme annulaire (82) comprend en outre une pluralité d'éléments d'espacement (110) formant des passages d'air supplémentaires communiquant entre ledit passage de fourniture d'air (56) et ledit passage de mélange (52).

3. Dispositif selon la revendication 1, comprenant en outre un élément d'injection de matériau de revêtement (58) comportant un tube (60) avec une sortie (60b), ledit tube (60) s'étendant à travers ledit passage central (62) avec ladite sortie positionnée dans ledit passage de mélange (52) ; et/ou comprenant en outre :
un élément de vanne (76) et un siège de vanne (78) montés dans ledit passage de fourniture de matériau de revêtement (74), ledit élément de vanne (76) étant mobile par rapport audit siège de vanne (78) pour fournir sélectivement le matériau de revêtement à la chambre allongée (30) ; et/ou
dans lequel ledit orifice (35) est configuré pour permettre à de l'air supplémentaire d'être attiré dans ladite chambre allongée (30) lorsque la brume revêt l'extérieur de l'objet allongé ; et/ou
dans lequel ladite chambre allongée (30) comporte un axe central (108) coaxial avec ledit orifice (35), et ledit passage de mélange s'étend de façon transversale par rapport à ladite chambre allongée ; et/ou
dans lequel l'objet allongé comprend en outre une aiguille creuse, et comprenant en outre :
un porte-aiguille (20) comportant un espace d'air (20a) et un élément de support d'aiguille (40) configuré pour fixer une première extrémité (36a) de l'aiguille (36) en communication avec l'espace d'air intérieur (20a) tandis qu'une deuxième extremité ouverte (36b) de l'aiguille s'étend vers l'extérieur depuis ledit porte-aiguille (20) pour l'insertion à travers ledit orifice (35) et dans ladite chambre allongée (30), dans lequel ledit espace d'air intérieur (20a) est adapté pour être pressurisé avec de l'air pour faire passer de l'air à travers l'aiguille (36) et empêcher l'obturation de la deuxième extrémité ouverte (36b) de l'aiguille (36) avec le matériau de revêtement ; et/ou
dans lequel ladite structure de forme annulaire (54) comporte un passage d'air formant une constriction dans ledit passage de mélange (52), entraînant ainsi un effet Venturi de l'air entrant dans ledit passage de mélange (52) ; et/ou
comprenant en outre :
un élément de vanne (76) et un siège de vanne (78) monté dans ledit passage de fourniture de matériau de revêtement (74), ledit élément de vanne (76) étant mobile par rapport audit siège de vanne (78) pour fournir sélectivement le matériau de revêtement à la chambre allongée (30) ; et/ou
dans lequel ledit orifice (35) est configuré pour permettre à de l'air supplémentaire d'être attiré dans ladite chambre allongée (30) lorsque la brume revêt l'extérieur de l'objet allongé.

4. Dispositif selon la revendication 3, dans lequel ladite structure de logement (12) comprend en outre un premier sous-logement (2) et un deuxième sous-logement (24), ledit premier sous-logement (22) étant couplé de façon amovible audit deuxième sous-logement (24) et contenant ledit passage de mélange (52), ladite structure de forme annulaire (54) et ladite chambre de revêtement allongée (30), et ledit deuxième sous-logement (24) contenant ledit élément de vanne (76) et ledit logement de vanne (78).

5. Procédé de revêtement d'une surface extérieure d'un objet allongé avec un matériau de revêtement en utilisant un dispositif (10) selon la revendication 1, comprenant :
le maintien de l'objet allongé dans le sens de la longueur dans une chambre allongée (30) ayant des première et deuxième extrémités opposées (32, 34) et une sortie à la deuxième extrémité,
le mélange d'air pressurisé et du matériau de revêtement pour former une brume; et le revêtement de la surface extérieure avec le matériau de revêtement tout en dirigeant la brume autour de la surface extérieure et vers la sortie de la chambre allongée (30).

6. Procédé selon la revendication 5, comprenant en outre :
le mélange de l'air pressurisé et du matériau de revêtement dans un passage de mélange (52) orienté transversalement dans la chambre allongée (30) avant de diriger l'air et le matériau de revêtement dans la chambre allongée (30),
dans lequel de préférence le passage de mélange (30) s'étend le long d'un axe (108) et le procédé comprend en outre :
le tourbillonnement de l'air autour de l'axe (108) à une entrée d'air dans le passage de mélange (52) ; et
la direction du matériau de revêtement à l'intérieur du passage de mélange (52) le long de l'axe central (108) pour mélanger avec l'air tourbillonnant.

7. Procédé selon la revendication 6, comprenant en outre :
la direction de l'air dans le passage de mélange (52) avec un effet Venturi.
